# FASCICULE DE BREVET EUROPEEN

(11) **EP 1 073 372 B1**
(45) Date de publication et mention de la délivrance du brevet: **22.12.2004**
(21) Numéro de dépôt: 99914632.7
(22) Date de dépôt: 21.04.1999
(51) Int. Cl.: A61B 17/04

(54) **ANCILLAIRE POUR LA MISE EN PLACE ET LE RETRAIT D'UN IMPLANT ET PLUS PARTICULIEREMENT D'UNE ANCRE DE SUTURE**
HILFSGERÄT ZUM EINSETZEN UND LÖSEN EINES IMPLANTATS, INSBESONDERE EINES CHIRURGISCHEN NÄHFADENANKERS
ANCILLARY FOR SETTING AND REMOVING AN IMPLANT AND MORE PARTICULARLY A SUTURE ANCHOR

(30) Priorité: 21.04.1998 FR 9805204
(43) Date de publication de la demande: 07.02.2001
(73) Titulaire: Tornier SA, 38330 Saint Ismier (FR)
(72) Inventeur: TORNIER, Alain, F-38330 Montbonnot (FR); BONNOMET, François, F-67000 Strasbourg (FR)
(74) Mandataire: Schmitt, John
(86) Numéro de dépôt international: PCT/FR1999/000939
(87) Numéro de publication internationale: WO 1999/053842

(56) Documents cités:
- US-A- 5 501 695
- US-A- 5 649 963

## Description

La présente invention est relative à un ancillaire, ou instrument, pour implant, et plus particulièrement pour une ancre de suture permettant de rattacher des tissus mous, tels que des ligaments ou tendons, à l'os pour la réparation de la coiffe des rotateurs et des lésions de Bankart.

On connaît d'après la demande EP 0 599 772, un ancillaire permettant la mise en place d'une ancre de suture dans l'os qui se présente sous la forme d'un pistolet. Ce dernier comporte une poignée susceptible d'être manipulée manuellement renfermant un mécanisme d'entraînement et une détente qui active ledit mécanisme. Le pistolet est solidaire d'un cylindre et d'un guide contenant une agrafe ou ancre de suture conformée pour recevoir un fil de suture. Le pistolet permet d'éjecter l'agrafe solidaire du fil de suture à l'extérieur du cylindre pour venir l'ancrer dans l'os pour diverses opérations médicales.

On note que l'extraction des ancres de suture comportant des moyens de fixation déformables est réalisée en perçant l'os cortical d'un trou dont le diamètre est légèrement supérieur à celui des moyens de fixation.

Dans ces conditions, le chirurgien dégrade considérablement la partie corticale de l'os, empêchant toute possibilité de reprise ou de mise en place d'une ancre de suture.

On connaît d'après le brevet EP 0 804 124, un dispositif pour la mise en place ou le retrait d'une ancre de suture à expansion.

Le dispositif de mise en place de l'ancre de suture comporte un outil qui se déplace en translation (traction) par rapport à un corps fixe pour déplacer une douille de compression qui vient déformer les moyens d'expansion de l'ancre de suture.

Le dispositif pour le retrait de l'ancre de suture comporte un outil qui se déplace en translation (compression) par rapport à un corps fixe pour déplacer l'ancre de suture à l'intérieur du trou dans lequel elle est engagée, afin de ramener les moyens d'expansion dans une direction sensiblement parallèle à celle de l'axe longitudinal de l'ancre de suture.

La déformation des moyens d'expansion provient uniquement de leurs caractéristiques élastiques, car le dispositif ne prévoit aucun moyen qui vient agir sur l'ancre de suture pour la déformer à nouveau, afin de l'extraire de l'os.

C'est à ces inconvénients qu'entend plus particulièrement remédier la présente invention.

L'ancillaire suivant la présente invention a pour objet la mise en place ou le retrait des ancres de suture à expansion réversible permettant la déformation des moyens d'expansion et le sertissage de fils de suture, ou la déformation de moyens de fixation lors d'opérations de réinsertion ligamentaire ou tendineuse réalisées sous arthroscopies ou à ciel ouvert.

L'ancillaire pour implant déformable élastiquement suivant la présente invention comprend un corps sur lequel s'articule une poignée mobile, des moyens de mouvements déplaçant une tige pour la déformation de l'implant et un dispositif de débrayage permettant, d'une part dans une première position débrayée, de laisser libre en rotation et en translation la tige par rapport au corps, et d'autre part dans une seconde position embrayée, de bloquer en rotation et en translation la tige par rapport au corps.

L'ancillaire de mise en place suivant un mode de réalisation préféré de l'invention comprend un corps sur lequel s'articule une poignée mobile, des moyens de traction déplaçant une tige de traction pour la déformation de l'implant, et un dispositif de débrayage permettant, d'une part dans une première position débrayée, de laisser libre en rotation et en translation la tige par rapport au corps, et d'autre part dans une seconde position embrayée, de bloquer en rotation et en translation la tige par rapport au corps.

L'ancillaire d'extraction suivant un mode de réalisation préféré de la présente invention comprend un corps sur lequel s'articule une poignée mobile, des moyens de compression déplaçant une tige de compression pour la déformation de l'implant et un dispositif de débrayage permettant de laisser, d'une part dans une première position débrayée, de laisser libre en rotation et en translation la tige par rapport au corps, et d'autre part dans une seconde position embrayée, de bloquer en rotation et en translation la tige par rapport au corps.

D'autres modes de réalisation préférés de l'invention sont définis dans les revendications 4-27.

La description qui va suivre en regard des dessins annexés, donnés à titre d'exemples non limitatifs, permettra de mieux comprendre l'invention, les caractéristiques qu'elle présente et les avantages qu'elle est susceptible de procurer :
Figure 1 est une coupe illustrant l'ancillaire suivant la présente invention.
Figure 2 est une coupe montrant l'ancillaire en position actionnée pour la déformation de l'implant.
Figure 3 est une vue de dessus montrant l'ancillaire suivant la présente invention.
Figure 4a et 4b sont des vues représentant en détail les embouts se fixant sur l'ancillaire afin de conformer les implants.
Figures 5a à 5c sont des vues illustrant la déformation d'un implant au moyen de l'ancillaire suivant la présente invention pour, d'une part sa fixation dans l'os, et d'autre part le sertissage de fil de suture.
Figures 6a à 6c sont des vues montrant une variante de l'ancillaire pour la conformation de la tête d'un implant en vue de la fixation de tissus mous contre l'os d'un patient.
Figure 7 est une coupe illustrant l'ancillaire suivant la présente invention.
Figure 8 est une vue de dessus montrant l'ancillaire suivant la présente invention.
Figure 9 est une coupe montrant l'ancillaire en position comprimée pour la déformation de l'implant en vue de son retrait de l'os.
Figure 10a à 10c sont des vues représentant en détail les étapes pour le retrait de l'implant par l'intermédiaire de l'ancillaire suivant la présente invention.

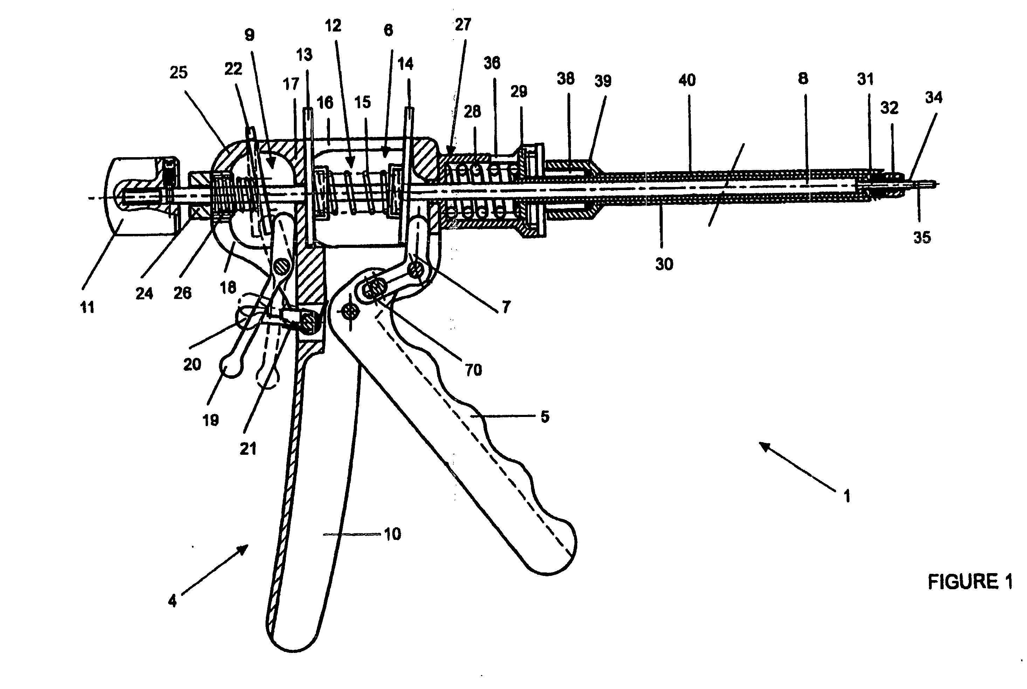

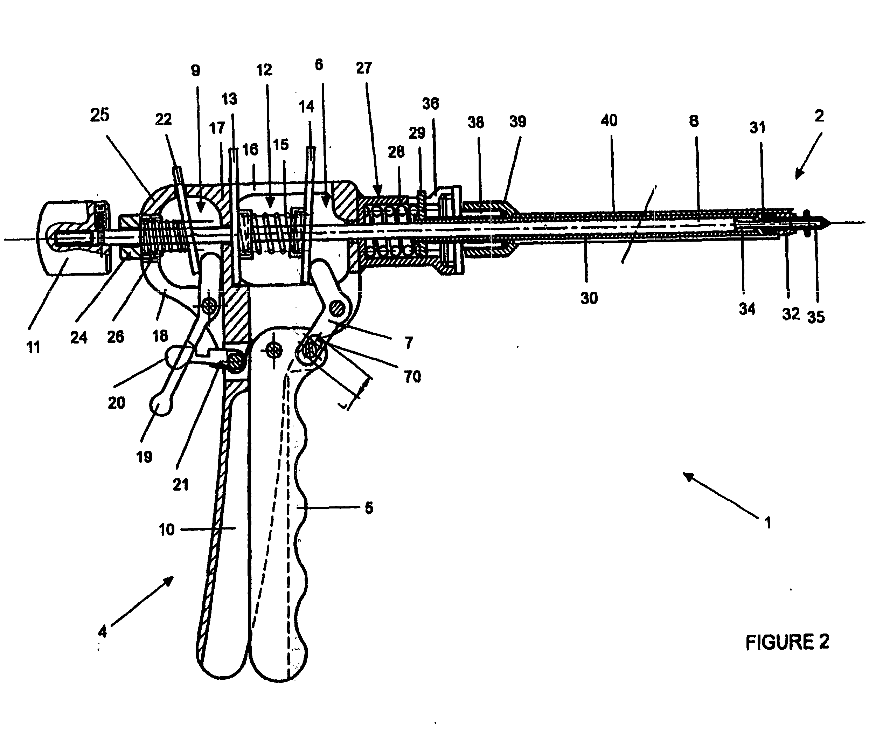

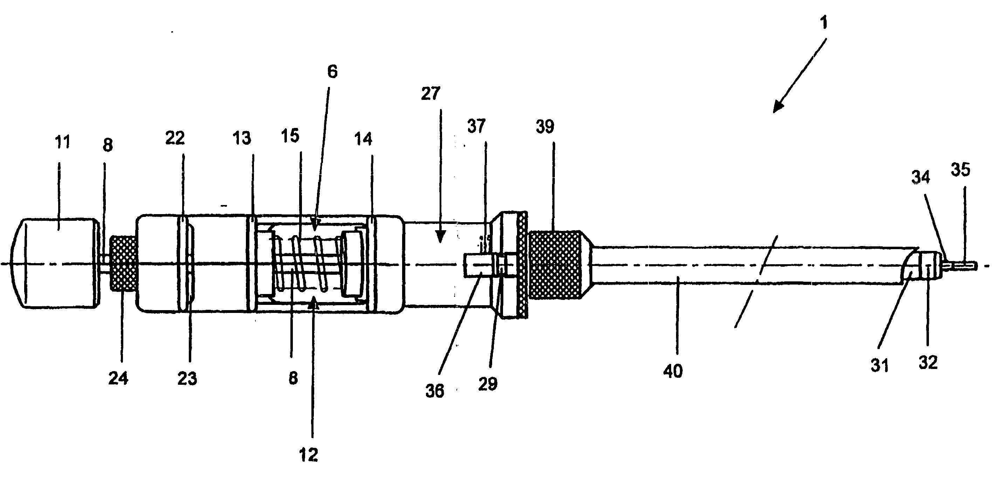

On a montré en figures 1 à 3 un ancillaire 1 permettant la mise en place d'implants, tels que des ancres de suture 2 et 3.

L'ancillaire 1 permet l'introduction et la fixation des ancres de suture 2 et 3 à expansion réversible dans l'os d'un patient.

L'ancillaire 1 permet également, lors de la fixation des ancres de sutures 2 et 3 dans l'os, la déformation des moyens d'expansion, des moyens de sertissage des fils de suture ou la déformation des moyens de fixation de l'ancre 3 lors d'opérations de réinsertion ligamentaire ou tendineuse réalisées sous arthroscopies ou à ciel ouvert.

L'ancillaire 1 comporte un corps 4 se présentant sous la forme d'un pistolet sur lequel vient s'articuler une poignée 5 susceptible de se déplacer sous un effort manuel. La poignée 5 est disposée en face d'une autre poignée fixe 10 conformée dans le corps 4 de l'ancillaire 1.

Le corps 4 présente une première chambre 6 dans laquelle débouche la poignée 5 par l'intermédiaire d'un renvoi coudé 7. La course de la poignée 5 est limitée par les dimensions que présente la boutonnière 70 ménagée dans le renvoi coudé 7. Cette chambre 6 est traversée horizontalement par une tige de traction 8 qui débouche également de part et d'autre du corps 4. La chambre 6 présente une forme oblongue munie d'une ouverture 16 sur le dessus du corps.

La chambre 6 reçoit de moyens de traction 12 qui sont constitués de deux plaques opposées 13, 14 entre lesquelles est comprimé un ressort 15. Les moyens de traction 12, et plus particulièrement les plaques 13 et 14 sont traversées par la tige de traction 8, tandis que le ressort 15 est enroulé coaxialement autour de ladite tige pour venir en contact avec chacune des plaques.

Les plaques 13 et 14 sont disposées perpendiculairement à l'axe longitudinal de la tige de traction 8 de manière que la seconde plaque 14 soit en contact avec le renvoi coudé 7 de la poignée mobile 5.

On note également que les plaques 13 et 14 débouchent à l'extérieur du corps 4 de l'ancillaire 1 par l'intermédiaire de l'ouverture 16 de la chambre 6.

La poignée mobile 5, sous un effort manuel, permet par l'intermédiaire du renvoi coudé 7, que la seconde plaque 14 s'arc-boute sur la tige de traction 8 sous l'effort de compression du ressort 15, tandis que la première plaque 13 reste toujours perpendiculaire à ladite tige et contre la paroi verticale 17 du corps 4 séparant les deux chambres 6 et 9.

Le corps 4 comporte une seconde chambre 9 disposée dans le prolongement de la première et du côté de la poignée fixe 10. La chambre 9 est traversée par la tige de traction 8 de manière que l'extrémité de cette dernière coopère avec une tête amovible 11.

La chambre 9 présente une ouverture inférieure 18 qui est tournée du côté de la poignée fixe 10 pour le passage d'un levier articulé 19.

Le levier 19 coopère avec un taquet de blocage 20 poussé par un ressort de torsion 21 qui est logé dans la poignée fixe 10.

Le levier 19 est en contact avec une plaque 22 qui est disposée autour de la tige de traction 8 et dans la seconde chambre 9. L'extrémité de la plaque 22, située à l'opposé de celle coopérant avec le levier 19, est logée dans une fente 23 ménagée dans le corps 4 et en face de l'ouverture 18.

L'introduction de la plaque 22 dans la fente 23 permet de constituer un point de pivotement de ladite plaque lorsqu'elle est soumise à un effort de basculement provenant du levier 19.

La chambre 9 est solidaire d'un embout 24 qui vient se visser dans la paroi verticale 25 opposée à celle 17 du corps 4. L'embout 24 est traversé par la tige de traction 8 pour la mise en place d'un ressort 26 qui est enroulé autour de ladite tige et comprimé entre ledit embout et la plaque 22. L'embout 24 permet le réglage de la tension du ressort 26.

Le dispositif décrit ci-dessus et monté dans la chambre 9 de l'ancillaire, constitue un système de débrayage permettant de laisser ou non la tige de traction 8 libre en rotation et en translation par rapport au corps 4.

En effet, la position débrayée de l'ancillaire 1 (traits pointillés sur la figure 1) est obtenue en appuyant sur le levier 19 jusqu'à ce qu'il vienne se bloquer sur le taquet 20 poussé par le ressort de torsion 21. Le basculement du levier 19 permet le déplacement de la plaque 22 suivant une position verticale, de manière à bloquer ladite plaque dans sa fente 23.

Dans cette position débrayée, la tige de traction 8 reste solidaire de la plaque 14, ce qui permet lorsqu'on comprime la poignée mobile 5 de déplacer ladite tige vers l'arrière du corps 4. Lorsqu'on relâche la poignée 5, le ressort 15, comprimé entre les plaques 13 et 14, permet de ramener la tige de traction 8 vers l'avant dans sa position d'origine.

La position débrayée de l'ancillaire 1 permet au chirurgien l'introduction de l'ancre de suture 2, 3 dans l'os, soit par vissage, soit par un effort de frappe qui est soumis à la tête amovible 11 solidaire de l'extrémité de la tige de traction 8. Lorsque l'ancre de suture 2, 3 est introduite par vissage dans l'os, la tête 11 est préalablement retirée pour permettre au chirurgien d'agir directement sur la tige de traction 8 pour l'entraîner en rotation. L'entraînement en rotation de la tige de traction 8 peut être réalisé manuellement par une poignée ou une perceuse, non représentée, qui vient se fixer sur ladite tige.

La position embrayée du dispositif de l'ancillaire 1 (traits forts sur la figure 1) est obtenue en appuyant sur le taquet 20 pour libérer le levier 19 afin que la plaque 22 vienne s'arc-bouter sur la tige de traction 8 sous l'effort du ressort 26.

Dans cette position embrayée la tige de traction 8 n'est plus libre ni en rotation, ni en translation par rapport au corps 4 de l'ancillaire 1.

Ainsi, la tige de traction 8 reste solidaire de la plaque 14, ce qui permet, lorsqu'on comprime la poignée mobile 5, de déplacer ladite tige vers l'arrière du corps 4. Lorsqu'on relâche la poignée 5 la tige de traction 8 reste bloquée dans cette position.

La position embrayée de l'ancillaire 1 permet au chirurgien, lorsque l'ancre de suture 2, 3 est préalablement introduite dans l'os, de déformer les moyens de fixation de ladite ancre pour la fixer dans l'os, et de la conformer pour fixer les tissus mous à réinsérer contre l'os.

Le corps 4 est solidaire dans le prolongement de la chambre 6 et à l'opposé de la seconde chambre 9, d'un nez 27 qui est traversé par la tige de traction 8. A l'intérieur du nez 27 est prévu autour de la tige 8, un ressort de compression 28 qui est en contact, d'un côté avec la paroi verticale dudit nez, et de l'autre côté avec une plaque verticale 29.

La plaque 29 est solidaire d'un tube 30 libre en translation et dans lequel coulisse la tige de traction 8 lorsqu'elle est soumise à un effort de traction. A l'opposé de la plaque 29 le tube 30 est solidaire d'un guide 31 comportant une partie interne filetée prévue pour recevoir, soit un embout 32, soit un embout 33 permettant respectivement la mise en place de l'ancre de suture 2 et 3.

La plaque 29 coopère avec une fente 36 ménagée dans la partie supérieure du nez 27, à proximité de laquelle sont gravés des repères 37 permettant de contrôler l'effort appliquer à l'ancre de suture 2, 3 pour sa déformation.

Le nez 27 est solidaire d'un manchon fixe 38 disposé coaxialement au tube 30 sur lequel vient se visser un outil 39 pour la déformation de l'ancre de suture 3 en vue de la fixation des tissus mous contre l'os.

L'outil 39 est constitué d'un tube 40, coaxial à celui 30, dont l'extrémité libre, disposé autour de l'embout 33 correspondant à la mise en place de l'ancre de suture 3, présente un profil courbe 41 pour permettre de plier les moyens de fixation de ladite ancre de suture.

La tige de traction 8 présente au niveau du guide 31 une extrémité 34 qui est d'un diamètre inférieur à celui prévu pour ladite tige et qui traverse soit l'embout 32, soit l'embout 33 suivant l'ancre de suture 2, 3 à fixer.

La portion de l'extrémité 34 de la tige de traction 8 se trouvant à l'extérieur de l'embout 32, 33 comporte une partie filetée 35 qui vient se visser à l'intérieur de l'ancre de suture 2, 3.

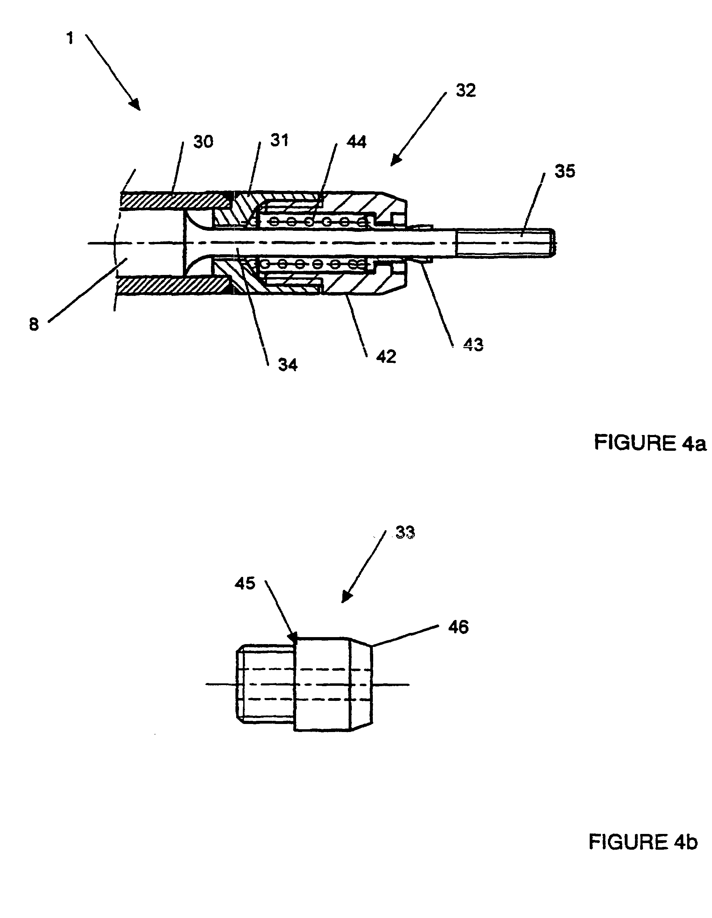

En figure 4a on a montré l'embout 32 permettant la mise en place de l'ancre de suture 2, lequel comprend un corps cylindrique 42 dont une partie vient se visser dans la partie interne du guide 31, tandis que l'autre partie vient en appui contre l'ancre de suture. La partie venant en appui contre l'ancre de suture est solidaire à proximité de la tige de traction 8 d'un guide-fil 43.

A l'intérieur du corps 42 est prévu un ressort 44 qui vient en appui contre le guide-fil 43, et la partie interne du guide 31 est solidaire du tube 30.

En figure 4b on a représenté l'embout 33 permettant la mise en place de l'ancre de suture 3, lequel comprend un corps cylindrique 45 dont une partie vient se visser dans la partie interne du guide 31, tandis que l'autre partie présente une face conique 46 qui vient en appui contre l'ancre de suture 3.

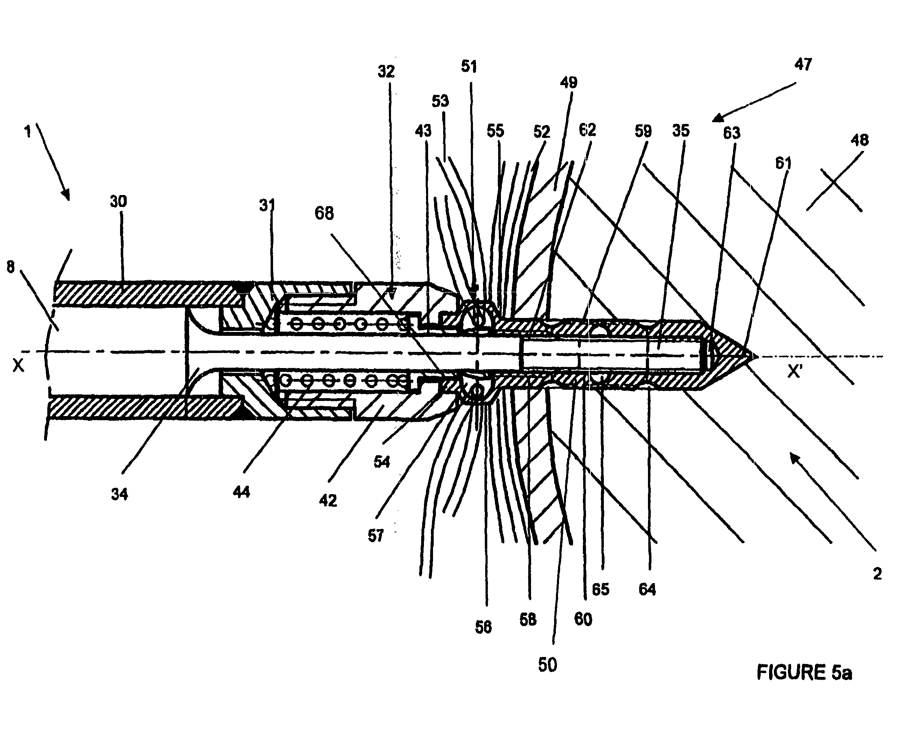

En figures 5a à 5c on a illustré la mise en place de l'ancre de suture 2 au moyen de l'ancillaire 1 dans l'os 47 d'un patient qui est constitué d'os spongieux 48 et d'os cortical 49.

L'ancre de suture 2 comporte un corps cylindrique creux 50 de forme allongée susceptible de se déformer plastiquement et pouvant être mis en place sur le site opératoire sous arthroscopie.

Le corps 50 comprend une tête 51 permettant la fixation des tissus mous 52 contre l'os 47 d'un patient par l'intermédiaire d'un ou plusieurs fils de suture 53 sertis sur ladite tête.

La tête 51 est constituée d'une face cylindrique d'appui 54 qui se prolonge par deux languettes 55, 56 opposées et parallèles à l'axe longitudinal XX' du corps 50, avant leur déformation. Les languettes 55 et 56 délimitent un espace oblong 57 qui permet le passage, par le chirurgien sur le site opératoire, d'au moins un fil de suture 53.

Les languettes 55 et 56 sont raccordées à l'opposé de la face 54 à une partie cylindrique 58 qui se prolonge par au moins deux branches de fixation 59, 60 qui sont, avant déformation, parallèles à l'axe longitudinal XX' du corps 50.

Les branches 59 et 60 se prolongent parallèlement à l'axe XX' par une pointe à profil conique 61 facilitant la mise en place de l'ancre de suture dans l'os 47.

La partie cylindrique 58 comporte un alésage interne fileté 62, porté par l'axe XX', qui débouche, d'une part entre les languettes 55, 56, et d'autre part entre les branches de fixation 59, 60.

Également, la pointe à profil conique 61 présente dans sa partie interne un trou borgne fileté 63 qui débouche entre les branches de fixation 59, 60 et qui est porté par le même axe longitudinal XX' que celui de l'alésage 62. En Outre le diamètre de l'alésage fileté 62 est prévu plus grand que celui du trou fileté 63.

Les branches 59 et 60 sont raccordées à la partie cylindrique 58 et à la pointe 61 par des amorces de pliage 64 dirigées en direction du centre du corps 50 et qui permettront de déformer lesdites branches sous un effort de traction appliqué par la tige 8 de l'ancillaire 1.

Les branches 59 et 60 présentent en leur milieu une amorce de pliage 65 disposée l'une en face de l'autre et en sens inverse pour constituer deux segments identiques et de même longueur.

Entre chaque branche 59 et 60 est prévue une butée solidaire de la partie cylindrique 58 et qui est dirigée en direction de la pointe à profil conique 61. Chaque butée, non représentée, s'étend parallèlement à l'axe longitudinal XX' du corps 50 et présente une longueur qui dépend de la déformation que l'on désire obtenir des branches 59 et 60.

En effet, la déformation des branches 59 et 60 est limitée par des butées, non représentées, qui viennent en appuie contre une face de la pointe à profil conique 61.

La face d'appui 54, réunissant les languettes 55, 56 à l'opposé de la partie cylindrique 58, est percée d'un alésage 68, coaxial à celui fileté 62, mais de plus grand diamètre. L'alésage 68 débouche entre les languettes 55, 56 et à l'intérieur de l'espace oblong 57.

La figure 5a montre, après introduction de l'ancre de suture 2 dans l'os 47, la tige de traction 8 visser dans le trou borgne 63 de la pointe à profil conique 61. Le guide 31 solidaire du tube 30 reçoit par vissage l'embout 32 de manière que le guide fil 43 pénètre par l'intermédiaire de l'alésage 68 à l'intérieur de l'espace oblong 57 délimité par les languettes 55, 56 de l'ancre 2.

On note que dans cette position, la face 54 de l'ancre de suture 2 est appuyée contre l'embout 32 de l'ancillaire 1. Egalement, le chirurgien positionne dans l'espace oblong 57 des fils de suture 53.

L'ancillaire 1 est placé par le chirurgien au moyen du levier 19 dans sa position embrayée de manière que la tige de traction 8 ne soit pas libre en translation et en rotation par rapport au corps 4.

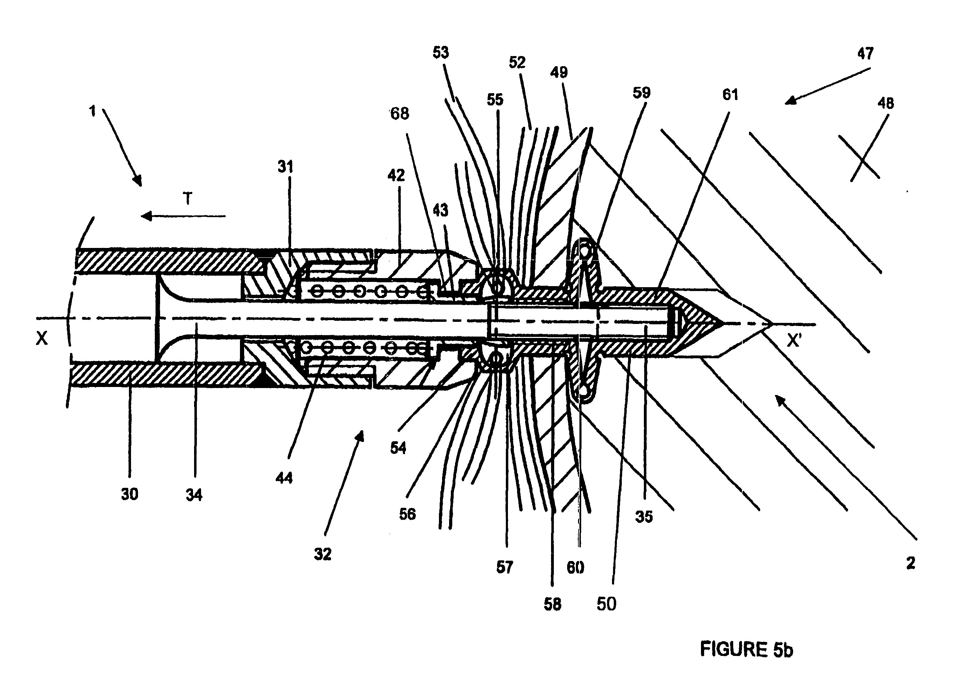

La figure 5b représente la déformation de l'ancre de suture 2 et plus particulièrement des branches 59 et 60 à l'intérieur de l'os spongieux 48 lorsqu'un effort de traction T est soumis à la tige 8. L'effort de traction T est engendré par des compressions successives de la poignée mobile 5 par le chirurgien afin de déplacer horizontalement suivant l'axe XX' la tige de traction 8 au moyen de la plaque 14 par rapport au corps 4.

Lors du déplacement de la tige 8 à l'intérieur du corps 4, on remarque que l'embout 32 reste fixe en appui contre la face 54 de l'ancre de suture 2.

La déformation des branches 59 et 60 est limitée jusqu'à ce que la pointe à profil conique 61 vienne en appui contre les butées, non représentées, du corps 50. Dès que la face de la pointe à profil conique 61 est en appui contre les butées, le chirurgien relâche la poignée 5. L'effort T appliqué à l'ancre de suture 2 est contrôlé par le déplacement de la plaque 29 par rapport aux repères 37 gravés sur le nez 27.

Les branches 59 et 60 se déforment sous un effort de compression du fait de la traction T soumise à la tige 8 de l'ancillaire 1 suivant le profil des amorces 64 et 65, de manière que les segments desdites branches soient dirigés à l'extérieur du corps 50 et dans une direction sensiblement perpendiculaire à l'axe XX'.

On note que la fixation de l'ancre de suture 2 dans l'os spongieux 48 est réalisée par la déformation des branches 59 et 60 jusqu'à ce que l'un des segments vienne en contact avec la face interne de l'os cortical 49.

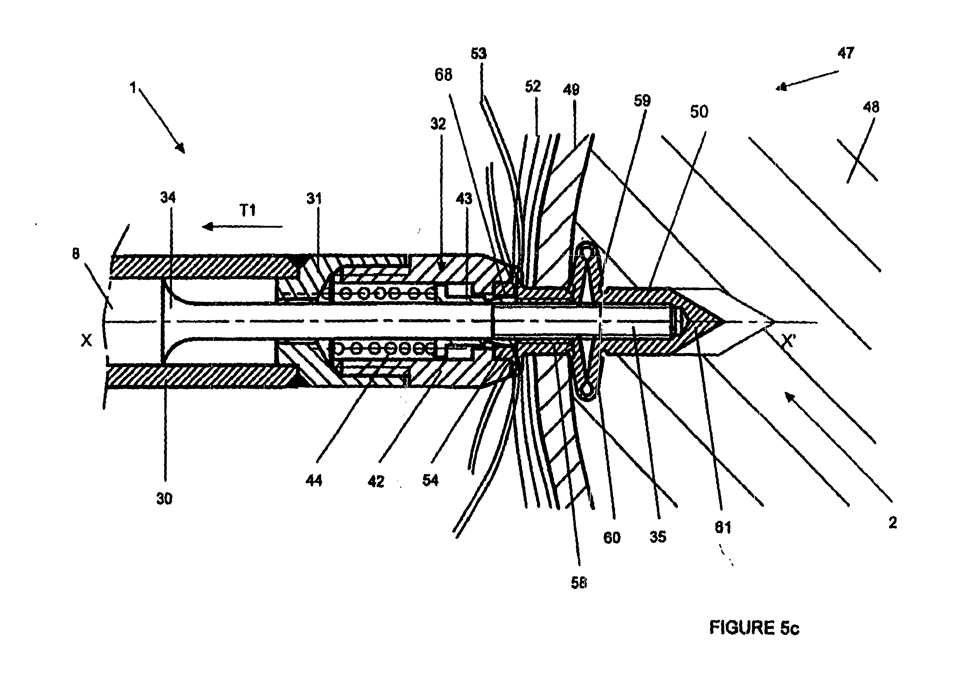

La figure 5c illustre le sertissage des fils de suture 53 sur la tête 51 de l'ancre 2 par l'intermédiaire de la déformation des languettes 55, 56.

La pointe à profil conique 61 étant en contact avec les butées, le chirurgien peut à nouveau comprimer la poignée 5 pour appliquer un effort de traction T1 plus important que celui T sur la tige de traction 8, sans risquer de rompre les branches 59 et 60, afin de déformer les languettes 55 et 56.

La déformation des languettes 55 et 56 permet de réduire l'espace oblong 57 de manière à bloquer dans une position tendue les fils de suture 53 sur la tête 51 de l'ancre de suture 2.

Lors de la déformation des languettes 55, 56, le guide-fil 43 permet d'éloigner les fils de suture 53 de la tige de traction 8 pour éviter de les endommager. Le guide-fil 43 est maintenu en contact à l'intérieur de l'ancre de suture 2 par le ressort 44 qui est indépendant du fonctionnement de l'ancillaire 1.

Les fils de suture 53 permettent au chirurgien de ligaturer les tissus mous 52 sur l'ancre de suture 2.

Après le sertissage des fils de suture 53, le chirurgien débraye l'ancillaire 1 pour dévisser la tige de traction 8 et la retirer de l'ancre de suture 2.

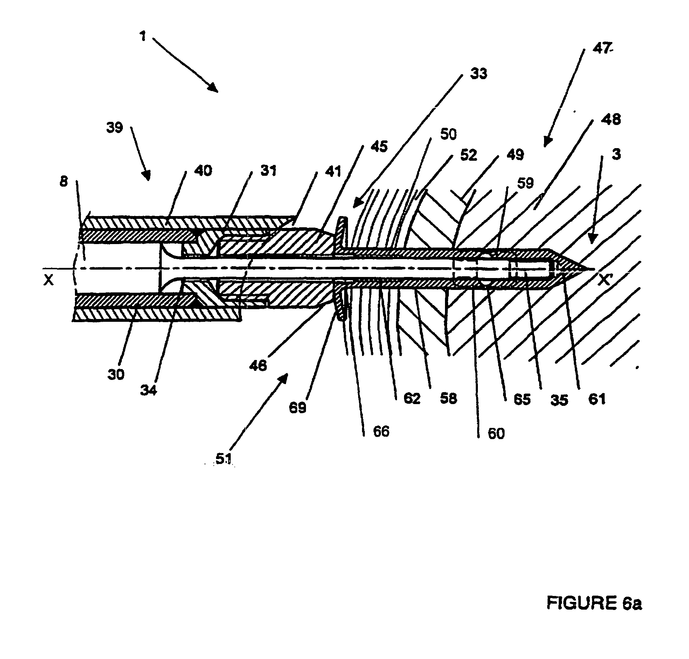

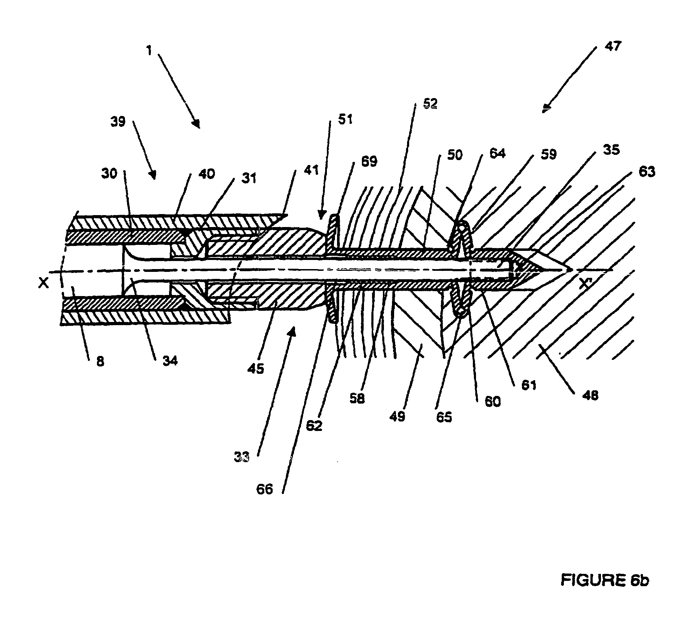

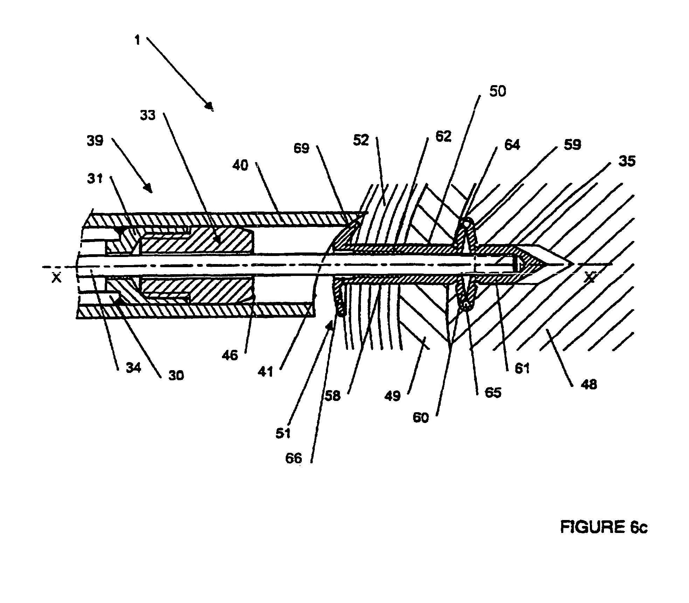

En figures 6a à 6c on a illustré la mise en place de l'ancre de suture 3 au moyen de l'ancillaire 1 dans l'os 47 d'un patient qui est constitué d'os spongieux 48 et d'os cortical 49.

L'ancre de suture 3 est semblable à celle 2, mais la tête 51 ne comporte pas les languettes 55 et 56 pour le sertissage des fils de suture 53.

La tête 51 est constituée de la partie cylindrique 58 plus longue que celle décrite précédemment et dans laquelle est percé l'alésage interne et fileté 62. Ce dernier débouche, d'une part entre les branches 59 et 60, et d'autre part à l'extérieur du corps 50 par l'intermédiaire d'un alésage coaxial 68 de plus grand diamètre.

La tête 51 comporte à l'extrémité de la partie cylindrique 58, et à l'opposé des branches 59 et 60, un disque 69 qui peut être conformé pour permettre la retenue des tissus mous 52 contre l'os 47.

Les étapes pour l'introduction et la fixation de l'ancre de suture 3 dans l'os 47 sont identiques à celles décrites précédemment pour l'ancre de suture 2 en figures 5a et 5b. On remarque que pour la mise en place de l'ancre de suture 3 dans l'os 47, l'ancillaire 1 comporte un embout 33 qui est vissé dans le guide 31 et un outil de mise en forme 39 qui est également vissé sur le nez 27.

Lorsque l'ancre de suture 3 est fixée dans l'os 47, et plus particulièrement dans l'os spongieux 48, c'est à dire que les branches 59 et 60 ont été déformées au moyen de la tige de traction 8 de l'ancillaire 1, le chirurgien débraye ladite tige 8 en appuyant sur le levier 19 pour amener la plaque 22 dans une position verticale (traits pointillés figure 1).

Cette position débrayée permet au chirurgien de reculer l'ancillaire 1 et l'embout 33 tout en laissant la tige de traction 8 vissée dans l'ancre de suture 3 (figure 6c).

Le chirurgien embraye à nouveau l'ancillaire 1 au moyen du levier 19 pour bloquer en translation et en rotation la tige de traction 8 par rapport au corps 4.

Le chirurgien amène l'outil de mise en forme 39 contre le disque 69 de la tête 51 en le dévissant légèrement du nez 27 (figure 6c).

Enfin, le chirurgien comprime par efforts successifs, la poignée 5 pour soumettre à l'outil de mise en forme 39 une force suffisante pour la déformation du disque 69 afin de bloquer les tissus mous 52 contre l'os cortical 49.

La conformation ou le pliage du disque 69 est réalisé en plusieurs fois par rotations successives de l'outil 39 autour de son axe pour présenter le profil courbe 41 dans une autre position. Le chirurgien procède de la même manière que ci-dessus pour conformer à nouveau le disque 69.

Après déformation du disque 69 le chirurgien débraye l'ancillaire 1 pour dévisser la tige de traction 8 et la retirer de l'ancre de suture 3.

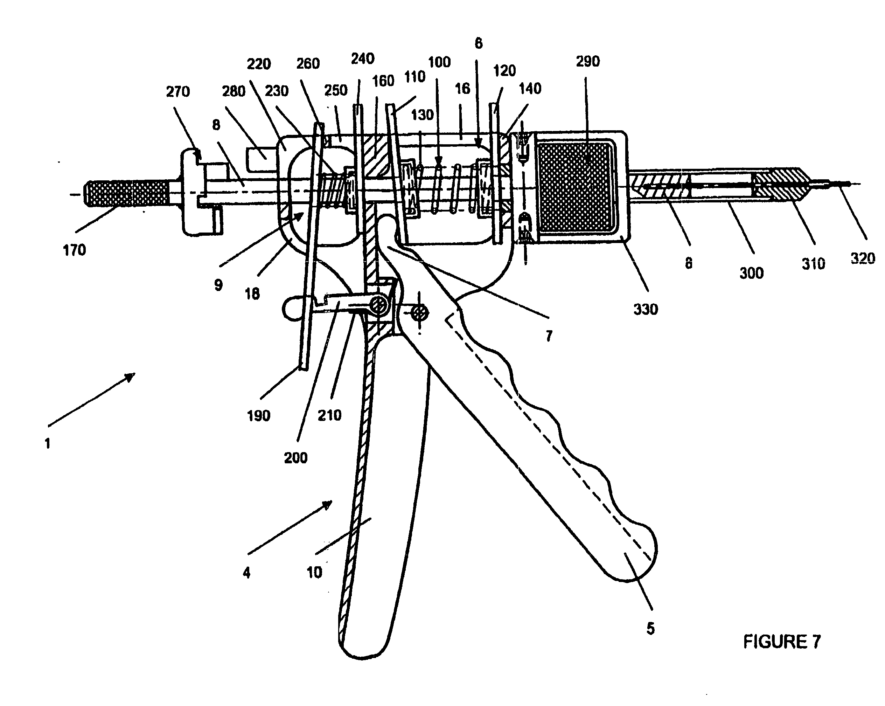

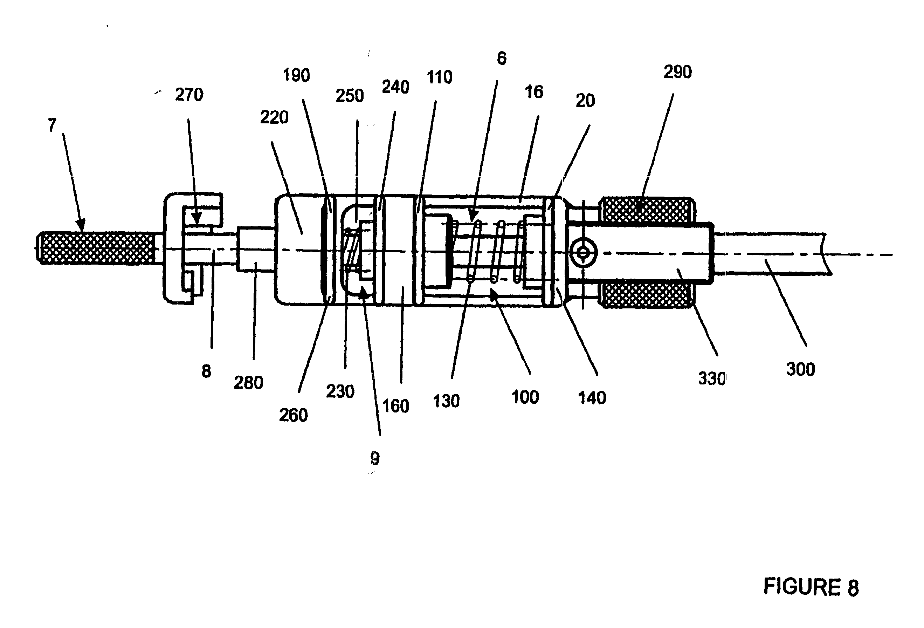

On a montré en figures 7 à 9 un ancillaire 1 permettant le retrait d'implants, tels que des ancres de suture 2, 3 décrites précédemment.

Par souci de clarté, nous avons gardé pour l'ancillaire d'extraction des références qui désignent des éléments qui sont identiques à ceux décrits pour l'ancillaire de mise en place. Seuls les éléments différents portent des références différentes.

L'ancillaire 1 permet le retrait de l'ancre de suture 2, 3 à expansion réversible dans l'os d'un patient par une déformation des moyens de fixation de ladite ancre pour les ramener dans une position sensiblement identique à celle d'origine.

Le corps 4 se présente sous la forme d'un pistolet sur lequel vient s'articuler la poignée 5 susceptible de se déplacer sous un effort manuel. La poignée 5 est disposée en face d'une autre poignée fixe 10 conformée dans le corps 4 de l'ancillaire 1.

Le corps 4 présente une première chambre 6 dans laquelle débouche la poignée 5 solidaire d'une extrémité en forme de renvoi coudé 7. Cette chambre 6 est traversée horizontalement par une tige 8 qui débouche également de part et d'autre du corps 4. La chambre 6 présente une forme oblongue munie d'une ouverture 16 sur le dessous du corps 4.

La chambre 6 reçoit des moyens de compression 100 de la tige 8 qui sont constitués de deux plaques opposées 110, 120 entre lesquelles est comprimé un ressort 130. Les moyens de compression 100, et plus particulièrement les plaques 110 et 120, sont traversées par la tige 8, tandis que le ressort 130 est enroulé coaxialement autour de ladite tige pour venir en contact avec chacune des plaques.

Les plaques 110 et 120 sont disposées sur la tige 8 de manière que la première plaque 110 soit en contact avec l'extrémité coudée 7 de la poignée mobile 5 suivant une direction inclinée par rapport à l'axe longitudinal de ladite tige.

On note également que les plaques 110 et 120 débouchent à l'extérieur du corps 4 de l'ancillaire 1 par l'intermédiaire de l'ouverture 16 de la chambre 6.

La poignée mobile 5, sous un effort manuel, permet à la première plaque 110 de s'arc-bouter sur la tige 8 en comprimant le ressort 130, tandis que la seconde plaque 120 reste toujours perpendiculaire à ladite tige et contre la paroi verticale 140 du corps 4 délimitant la chambre 6.

Le corps 4 comporte une seconde chambre 9 disposée dans le prolongement de la première 6 et du côté opposé à la paroi verticale 140. La chambre 9 est séparée de la chambre 6 par une paroi verticale 160 se trouvant dans le prolongement de la poignée fixe 10 du corps 4. La chambre 9 est traversée longitudinalement par la tige 8 de manière que l'extrémité de cette dernière se trouvant à l'extérieur du corps 4 et derrière la poignée fixe 10, coopère avec une tête moletée 170.

La tête moletée 170 présente des échancrures 270 de dimensions différentes qui coopèrent avec une butée 280 située dans la partie supérieure et extérieure de la chambre 9, pour permettre à la tige 8 d'effectuer une course plus ou moins importante en fonction du modèle de l'implant ou ancre de suture 2, 3 à retirer.

La chambre 9 présente une ouverture inférieure 18 qui est tournée du côté de la poignée fixe 10 pour le passage d'une plaque 190.

La plaque 190 coopère au niveau de l'une de ses extrémités avec un taquet de blocage 200 poussé par un ressort de torsion 210 qui est logé dans la poignée fixe 10.

La plaque 190 est disposée autour de la tige 8 et dans la seconde chambre 9 de manière que l'autre extrémité située à l'opposé de celle coopérant avec le taquet 200, soit logée dans une fente 220 ménagée dans le corps 4.

La plaque 190 est reliée par l'intermédiaire d'un ressort 230 enroulé autour de la tige 8 à une autre plaque 240 qui est en appui contre la paroi verticale 160. La plaque 240 débouche dans une autre fente supérieure 250 ménagée dans le corps 4 de l'ancillaire 1.

Les fentes 220 et 250 sont séparées par une cloison 260 sur laquelle vient prendre appui la plaque 190 suivant une direction inclinée par rapport à l'axe longitudinal de la tige 8.

Le dispositif décrit ci-dessus et monté dans la chambre 9 de l'ancillaire 1, constitue un système de débrayage permettant de laisser ou non la tige 8 libre en rotation et en translation par rapport au corps 4.

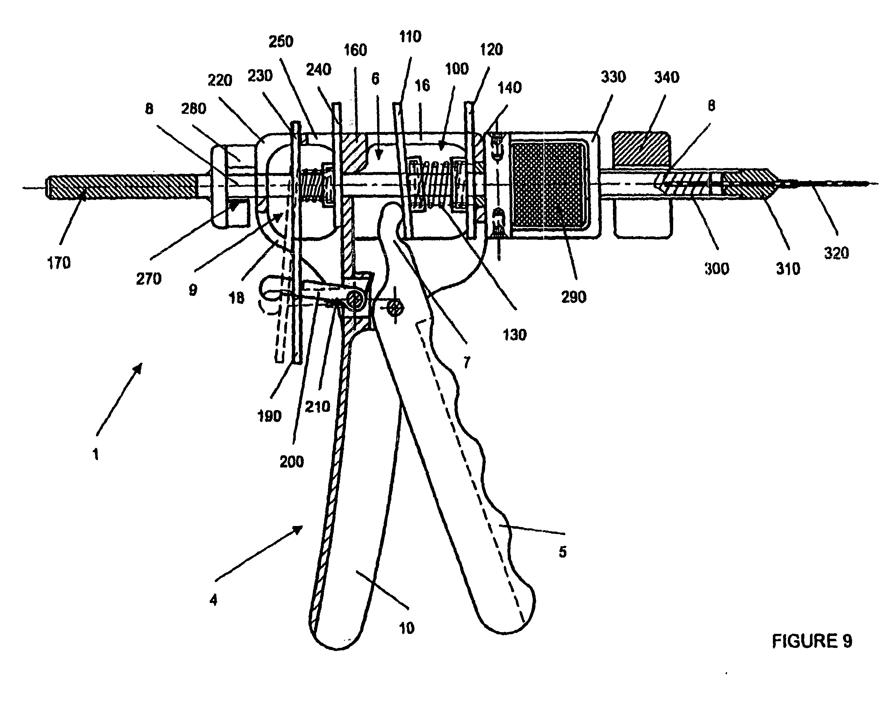

En effet, la position débrayée de l'ancillaire 1 (traits forts figure 9) est obtenue en appuyant sur la plaque 190 jusqu'à ce qu'elle vienne se bloquer sur le taquet 200 poussé par le ressort de torsion 210.

On remarque que dans cette position la plaque 190 présente une position sensiblement verticale sous l'effet de la poussée du ressort 230, de l'appui contre la cloison 260 et du niveau de blocage de la plaque 190 sur le taquet 200.

Dans cette position débrayée, la tige 8 est libre en rotation et en translation par rapport au corps 4 de manière à pouvoir la manipuler indépendamment de l'ancillaire 1.

La position embrayée du dispositif de l'ancillaire 1 (traits forts sur la figure 7) est obtenue en appuyant sur le taquet 200 pour libérer la plaque 190 afin qu'elle vienne s'arc-bouter sur la tige 8 sous l'effort du ressort 230.

Dans cette position embrayée, la tige 8 n'est plus libre ni en rotation, ni en translation par rapport au corps 4 de l'ancillaire 1.

Ainsi, la tige 8 reste solidaire de la plaque 110, ce qui permet lorsqu'on comprime la poignée mobile 5, de déplacer ladite tige vers l'avant du corps 4. Lorsqu'on relâche la poignée 5 la tige 8 reste bloquée dans cette position.

La position embrayée permet au chirurgien le retrait de l'ancre de suture 2, 3 en appliquant un effort suffisant pour déformer les moyens de fixation de ladite ancre pour qu'ils reviennent dans une position sensiblement équivalente à celle d'origine.

Le corps 4 est solidaire dans le prolongement de la chambre 6 et à l'opposé de la seconde chambre 9 d'un élément moleté 290 solidaire d'une gaine 300 dans laquelle coulisse la tige 8. La gaine 300 est solidaire à son extrémité libre d'un embout fileté 310 qui est traversé longitudinalement par une partie amincie 320 formant poussoir de la tige 8.

L'élément moleté 290 est protégé partiellement par une cage 330 qui est solidaire du corps 4 dans le prolongement de la chambre 6.

On note également qu'une masselotte mobile 340 vient s'adapter sur le profil extérieur de la gaine 300 pour permettre au chirurgien d'extraire l'ancre de suture 2 en appliquant quelques à-coups contre la cage 330.

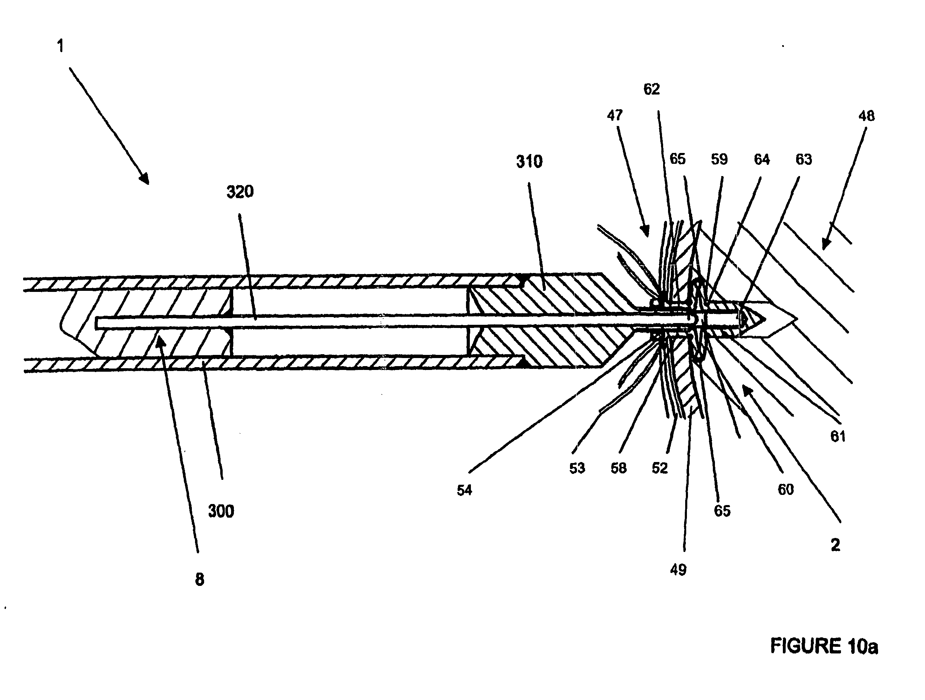

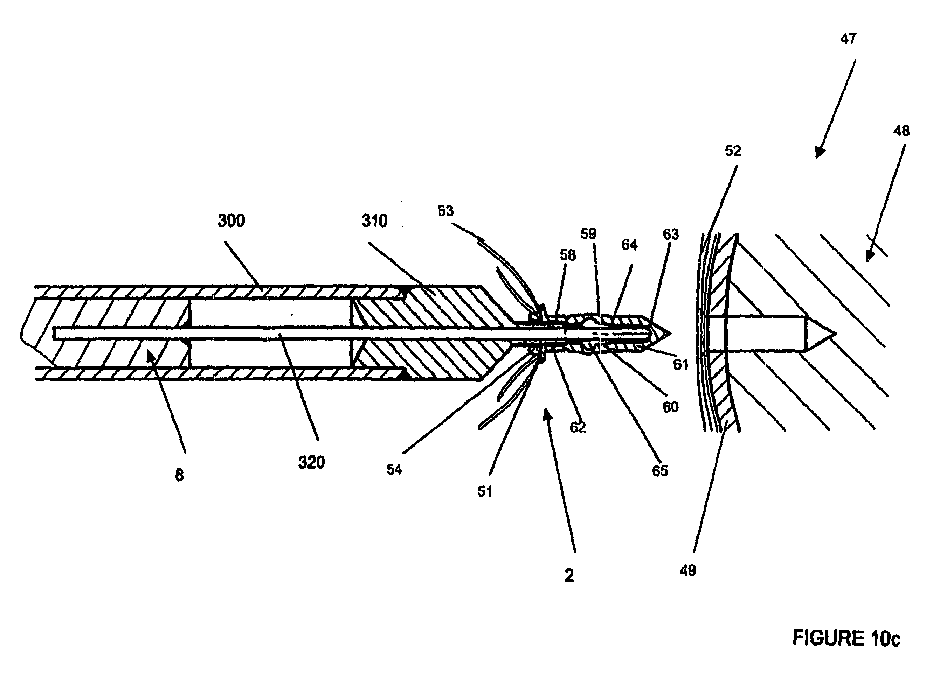

En figures 10a à 10c, on a illustré les différentes étapes de l'ancillaire pour extraire l'ancre de suture 2 de l'os 47 qui a été décrite précédemment. Celui-ci permet l'extraction de l'ensemble des ancres de suture ayant des moyens de fixation réversibles.

La figure 10a montre l'introduction de l'ancillaire 1 sur le site opératoire sous arthroscopie. Préalablement à la mise en place de l'ancillaire 1, le chirurgien vérifie le modèle de l'ancre de suture 2 à retirer, pour pouvoir indexer, par rotation de la tige 8 l'échancrure 270 correspondante de la tête moletée 170, afin qu'elle se trouve en face de la butée 280 solidaire du corps 4.

Le réglage de l'échancrure 270 permet de limiter la course de la tige 8 pour éviter toute rupture de l'ancre de suture à extraire à l'intérieur de l'os.

Ensuite, le chirurgien vient visser l'embout 310 de l'ancillaire 1 en position débrayée, par l'intermédiaire de l'élément moleté 290 à l'intérieur de l'ancre de suture 2, et plus particulièrement dans l'alésage fileté 62. Le chirurgien amène le poussoir 320 de la tige 8 dans le fond du trou borgne 63 ménagé dans la pointe à profil conique 61.

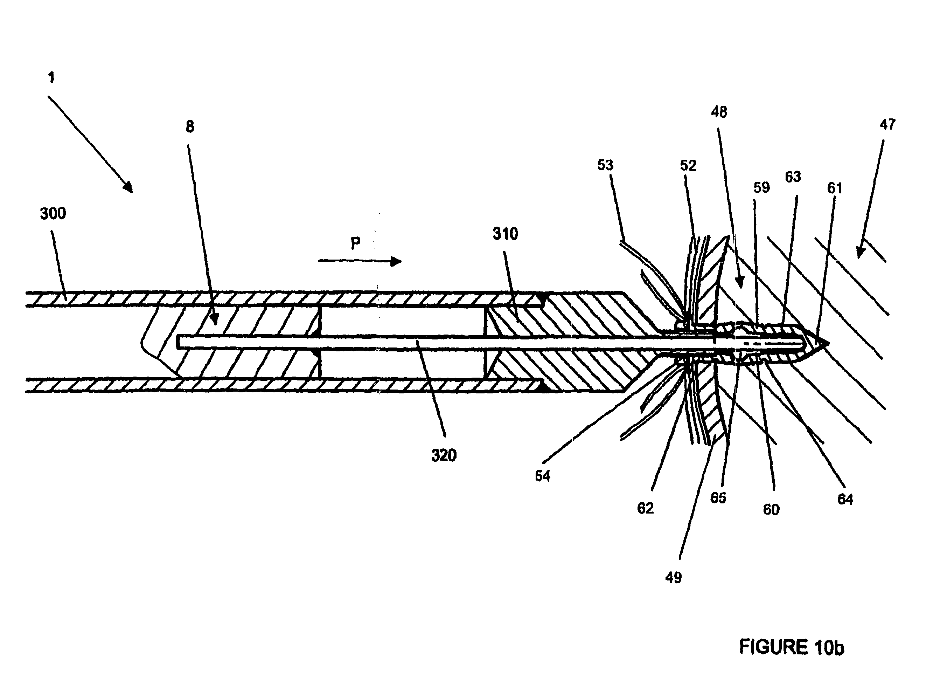

A la figure 10b, le chirurgien embraye l'ancillaire 1 au moyen de la plaque 190 pour pouvoir agir sur la tige 8 en comprimant la poignée mobile 5. La tige 8, à l'aide de son poussoir 320, applique un effort de pousser P sur la pointe à profil conique 61 pour déplier les branches 59, 60 et les amener dans une position sensiblement parallèle à l'axe XX' du corps 50 de l'ancre de suture 2.

Dès que l'ancre de suture 2 a retrouvé une position sensiblement allongée, le chirurgien peut à l'aide de la masselotte 340 taper sur la cage 330 de l'ancillaire 1 pour extraire ladite ancre de l'os 47 (figure 10c).

L'ancillaire 1 permet l'extraction des ancres de suture 2 ou analogues sans à avoir à percer un trou dont le diamètre serait sensiblement voisin de celui affecté par les branches déformées 59, 60.

On note que l'ancillaire 1 pour le retrait permet aussi bien le retrait de l'ancre de suture 2 que celle 3 montrée et décrite précédemment pour l'ancillaire de mise en place.

## Revendications

1. Ancillaire pour implant (2, 3) déformable élastiquement comprenant un corps (4) sur lequel s'articule une poignée mobile (5) et des moyens de mouvements (7, 14, 15 ; 110, 120, 130) déplaçant une tige (8) pour la déformation de l'implant (2, 3), **caractérisé en ce qu**'il comprend par ailleurs un dispositif de débrayage (19, 20, 21, 22, 26 ; 190, 200, 210, 230) permettant, d'une part dans une première position débrayée, de laisser libre en rotation et en translation la tige (8) par rapport au corps (4), et d'autre part dans une seconde position embrayée, de bloquer en rotation et en translation la tige (8) par rapport au corps (4).

2. Ancillaire pour la mise en place d'un implant (2, 3) déformable élastiquement pour sa fixation dans l'os (47) d'un patient suivant la revendication 1, **caractérisé en ce qu'il** comporte des moyens de traction (7, 14, 15) déplaçant la tige (8) pour la déformation de l'implant (2, 3).

3. Ancillaire d'extraction pour le retrait des implants (2, 3) de l'os (47) suivant la revendication 1, **caractérisé en ce qu**'il comprend des moyens de compression (110, 120, 130) déplaçant la tige (8) pour la déformation de l'implant (2).

4. Ancillaire pour la mise en place suivant la revendication 2, **caractérisé en ce que** le corps (4) comporte des moyens de contrôle (27, 28, 29) de l'effort de traction appliqué à la tige (8) pour la déformation de l'implant (2, 3).

5. Ancillaire pour la mise en place suivant la revendication 2, **caractérisé en ce que** le corps (4) comporte une première chambre (6) dans laquelle sont logés les moyens de traction (7, 14, 15), et une seconde chambre (9) recevant le dispositif de débrayage (19, 20, 21, 22, 26).

6. Ancillaire pour la mise en place suivant la revendication 2, **caractérisé en ce que** les moyens de traction sont constitués d'une première plaque (13) et d'une seconde plaque (14) entre lesquelles est comprimé un ressort (15) de sorte que la seconde plaque (14) coopère avec un renvoi coudé (7) actionné par la poignée mobile (5) pour arc-bouter ladite seconde plaque (14) sur la tige de traction (8) afin de la faire reculer par rapport au corps (4).

7. Ancillaire pour la mise en place suivant la revendication 2, **caractérisé en ce que** le dispositif de débrayage comprend un levier (19) qui est susceptible d'être bloqué par un taquet (20) chargé élastiquement sur une poignée fixe (10) du corps (4), une plaque (22) reliée à un ressort (26) dont la tension est réglable afin que ladite plaque puisse s'arc-bouter sur la tige de traction (8) sous un effort dudit levier pour passer d'uné position verticale débrayée à une position inclinée embrayée.

8. Ancillaire pour la mise en place suivant la revendication 4, **caractérisé en ce que** les moyens de contrôle sont constitués d'un nez (27) solidaire du corps (4) dans lequel est prévu autour de la tige (8) un ressort de compression (28), une plaque (29) solidaire d'un tube (30) libre en translation et dans lequel coulisse ladite tige.

9. Ancillaire pour la mise en place suivant la revendication 8, **caractérisé en ce que** la plaque (29) débouche dans une fente (36) ménagée dans la partie supérieure du nez (27) et à proximité de laquelle sont gravés des repères (37) permettant de contrôler l'effort appliqué à l'implant (2, 3).

10. Ancillaire pour la mise en place suivant la revendication 8, **caractérisé en ce que** le tube (30) présente à son extrémité libre un guide (31) comportant une partie interne filetée prévue pour recevoir un embout (32, 33) permettant la conformation de l'implant (2, 3).

11. Ancillaire pour la mise en place suivant la revendication 2, **caractérisé en ce que** la tige de traction (8) traverse de part en part le corps (4) de manière que son extrémité se trouvant du côté du dispositif de débrayage coopère avec une tête amovible (11) permettant l'introduction de l'implant (2, 3) dans l'os (47), tandis que l'autre extrémité (34) présente un diamètre inférieur à celui de ladite tige et une partie filetée (35) qui est adaptée pour venir se visser à l'intérieur de l'implant (2, 3).

12. Ancillaire pour la mise en place suivant la revendication 10, **caractérisé en ce que** l'embout (32) comprend un corps cylindrique (42) dont une partie est adaptée pour venir se visser dans le guide (31), tandis que l'autre partie vient s'appuyer contre l'implant (2).

13. Ancillaire pour la mise en place suivant la revendication 12, **caractérisé en ce que** l'embout (32) comporte dans sa partie interne un ressort (44) qui agit en compression sur un guide-fil (43).

14. Ancillaire pour la mise en place suivant la revendication 10, **caractérisé en ce que** l'embout (32) comprend un corps cylindrique (45) dont une partie coopère avec le guide (31) et l'autre partie présente une face conique (46) qui est adaptée pour venir en appui contre l'implant (3).

15. Ancillaire pour la mise en place suivant la revendication 10, **caractérisé en ce que** l'embout (32, 33) est traversé par la tige de traction (8) pour que l'extrémité (34) soit adaptée pour venir se visser dans l'implant (2, 3).

16. Ancillaire pour la mise en place suivant la revendication 8, **caractérisé en ce que** le nez (27) reçoit par vissage un outil de mise en forme (39) coaxial au tube (30) pour conformer l'implant (3).

17. Ancillaire pour la mise en place suivant la revendication 16, **caractérisé en ce que** l'outil (39) présente du côté du guide (31) un profil courbe (41).

18. Ancillaire d'extraction suivant la revendication 3, **caractérisé en ce que** le corps (4) comporte des moyens d'ancrage (290, 300, 310) libres en rotation autour de la tige (8) qui sont adaptés pour venir se fixer à l'intérieur de l'implant (2) à extraire.

19. Ancillaire d'extraction suivant la revendication 3, **caractérisé en ce que** le corps (4) comporte une première chambre (6) dans laquelle sont logés les moyens de compression, et une seconde chambre (9) recevant le dispositif de débrayage.

20. Ancillaire d'extraction suivant la revendication 3, **caractérisé en ce que** les moyens de compression sont constitués d'une première plaque (110) et d'une seconde plaque (120) entre lesquelles est comprimé un ressort (130), ladite première plaque (110) coopérant avec la poignée mobile (5) pour l'arc-bouter sur la tige (8) afin de la faire avancer par rapport au corps (4).

21. Ancillaire d'extraction suivant la revendication 3, **caractérisé en ce que** le dispositif de débrayage comprend une plaque (190) qui est susceptible d'être bloquée par un taquet (200) chargé élastiquement sur la poignée fixe (10) du corps (4), ladite plaque venant s'arc-bouter sur la tige (8) par l'intermédiaire d'un ressort (230) solidaire d'une autre plaque verticale (240) traversée par ladite tige, la plaque (190) pouvant prendre une position verticale débrayée à une position inclinée embrayée.

22. Ancillaire d'extraction suivant la revendication 18, **caractérisé en ce que** les moyens d'ancrage sont constitués d'un élément moleté (290) solidaire d'une gaine (300) portant à son extrémité libre un embout fileté (310) qui est adapté pour venir se visser à l'intérieur de l'implant (2).

23. Ancillaire d'extraction suivant la revendication 19, **caractérisé en ce que** les moyens d'ancrage sont constitués d'un élément moleté (290) solidiare d'une gaine (300) portant à son extrémité libre un embout filité (310) qui est adapté pour venir se visser à l'intérieur de l'implant (2), et **en ce que** l'élément moleté (290) est fixé sur le corps (4) dans le prolongement de la première chambre (6) à l'opposé de la seconde chambre (9).

24. Ancillaire d'extraction suivant la revendication 22, **caractérisé en ce que** l'élément moleté (290) est protégé partiellement par une cage (330) solidaire du corps (4).

25. Ancillaire d'extraction suivant la revendication 19, **caractérisé en ce que** la tige (8) traverse de part en part le corps (4) pour déboucher à l'extérieur du côté de la seconde chambre (9).

26. Ancillaire d'extraction suivant la revendication 22, **caractérisé en ce que** la tige (8) comporte une partie amincie (320) formant poussoir qui coulisse à l'intérieur de l'embout fileté (310) des moyens d'ancrage.

27. Ancillaire d'extraction suivant la revendication 3, **caractérisé en ce que** la tige (8) comporte une tête moletée (170) pourvue d'échancrures (270) de dimensions différentes, dont une au moins vient en contact avec une butée (280) solidaire du corps (4), après réglage en rotation de ladite tige pour limiter sa course en translation.

## Claims

1. Ancillary for an elastically deformable implant (2, 3), comprising a body (4) on which a movable grip (5) is articulated, and movement means (7, 14, 15; 110, 120, 130) displacing a rod (8) for deformation of the implant (2, 3), **characterized in that** it also comprises a disengaging device (19, 20, 21, 22, 26; 190, 200, 210, 230) with which it is possible, on the one hand in a first, disengaged position, for the rod (8) to be free in rotation and in translation relative to the body (4), and, on the other hand in a second, engaged position, for the rod (8) to be blocked in rotation and in translation relative to the body (4).

2. Ancillary according to Claim 1 for positioning of an elastically deformable implant (2, 3) for its fixation in the bone (47) of a patient, **characterized in that** it comprises traction means (7, 14, 15) displacing the rod (8) for deformation of the implant (2, 3).

3. Ancillary according to Claim 1 for withdrawing the implants (2, 3) from the bone (47), **characterized in that** it comprises compression means (110, 120, 130) displacing the rod (8) for deformation of the implant (2).

4. Ancillary according to Claim 2 for positioning of an implant (2, 3), **characterized in that** the body (4) comprises means (27, 28, 29) for controlling the tensile force applied to the rod (8) for deformation of the implant (2, 3).

5. Positioning ancillary according to Claim 2, **characterized in that** the body (4) comprises a first chamber (6) in which the traction means (7, 14, 15) are housed, and a second chamber (9) receiving the disengaging device (19, 20, 21, 22, 26).

6. Positioning ancillary according to Claim 2, **characterized in that** the traction means consist of a first plate (13) and of a second plate (14) between which a spring (15) is compressed in such a way that the second plate (14) cooperates with a bent countershaft (7) actuated by the movable grip (5) so as to brace said second plate (14) on the traction rod (8) in order to cause it to move back relative to the body (4).

7. Positioning ancillary according to Claim 2, **characterized in that** the disengaging device comprises a lever (19) which can be blocked by an elastically loaded catch (20) on a fixed grip (10) of the body (4) and a plate (22) connected to a spring (26) whose tension is adjustable so that said plate can be braced on the traction rod (8) under the effect of a force of said lever in order to pass from a disengaged vertical position to an engaged inclined position.

8. Positioning ancillary according to Claim 4, **characterized in that** the control means consist of a nose (27) which is integral with the body (4) and in which a compression spring (28) is provided around the rod (8), and a plate (29) integral with a tube (30) which is free in translation and in which said rod slides.

9. Positioning ancillary according to Claim 8, **characterized in that** the plate (29) opens into a slit (36) which is formed in the upper part of the nose (27) and in the proximity of which reference marks (37) are etched, making it possible to control the force applied to the implant (2, 3).

10. Positioning ancillary according to Claim 8, **characterized in that** the tube (30) has, at its free end, a guide (31) with an internal threaded part intended to receive a ferrule (32, 33) permitting shaping of the implant (2, 3).

11. Positioning ancillary according to Claim 2, **characterized in that** the traction rod (8) passes right through the body (4) in such a way that its end located towards the disengaging device cooperates with a removable head (11) permitting introduction of the implant (2, 3) into the bone (47), while the other end (34) has a diameter smaller than that of said rod and a threaded part (35) designed to be screwed inside the implant (2, 3).

12. Positioning ancillary according to Claim 10, **characterized in that** the ferrule (32) comprises a cylindrical body (42) of which one part is designed to be screwed into the guide (31), while the other part bears against the implant (2).

13. Positioning ancillary according to Claim 12, **characterized in that** the ferrule (32) comprises, on its inside, a spring (44) which acts by compression on a thread guide (43).

14. Positioning ancillary according to Claim 10, **characterized in that** the ferrule (32) comprises a cylindrical body (45) of which one part cooperates with the guide (31) and the other part has a conical face (46) designed to bear against the implant (3).

15. Positioning ancillary according to Claim 10, **characterized in that** the ferrule (32, 33) is traversed by the traction rod (8) so that the end (34) is designed to be screwed into the implant (2, 3).

16. Positioning ancillary according to Claim 8, **characterized in that** the nose (27) receives a shaping tool (39) which is screwed in coaxially with respect to the tube (30) for shaping the implant (3).

17. Positioning ancillary according to Claim 16, **characterized in that** the tool (39) has a curved profile (41) on the side towards the guide (31).

18. Withdrawal ancillary according to Claim 3, **characterized in that** the body (4) comprises anchoring means (290, 300, 310) which are free in rotation around the rod (8) and are designed to be fixed inside the implant (2) that is to be withdrawn.

19. Withdrawal ancillary according to Claim 3, **characterized in that** the body (4) comprises a first chamber (6) in which the compression means are housed, and a second chamber (9) receiving the disengaging device.

20. Withdrawal ancillary according to Claim 3, **characterized in that** the compression means consist of a first plate (110) and of a second plate (120) between which a spring (130) is compressed, said first plate (110) cooperating with the movable grip (5) in order to brace it on the rod (8) so as to make it move forwards relative to the body (4).

21. Withdrawal ancillary according to Claim 3, **characterized in that** the disengaging device comprises a plate (190) which can be blocked by an elastically loaded catch (200) on the fixed grip (10) of the body (4), said plate being braced on the rod (8) by way of a spring (230) formed integrally with another vertical plate (240) through which said rod passes, the plate (190) being able to assume a disengaged vertical position to an engaged inclined position.

22. Withdrawal ancillary according to Claim 18, **characterized in that** the anchoring means consist of a knurled element (290) formed integrally with a sleeve (300) which, at its free end, has a threaded ferrule (310) designed to be screwed inside the implant (2).

23. Withdrawal ancillary according to Claim 19, **characterized in that** the anchoring means consist of a knurled element (290) formed integrally with a sleeve (300) which, at its free end, has a threaded ferrule (310) designed to be screwed inside the implant (2), and **in that** the knurled element (290) is fixed to the body (4) in the continuation of the first chamber (6) directed away from the second chamber (9).

24. Withdrawal ancillary according to Claim 22, **characterized in that** the knurled element (290) is partially protected by a cage (330) formed integrally with the body (4).

25. Withdrawal ancillary according to Claim 19, **characterized in that** the rod (8) passes right through the body (4) and opens to the outside from the second chamber (9).

26. Withdrawal ancillary according to Claim 22, **characterized in that** the rod (8) comprises a thinner part (320) forming a pusher which slides inside the threaded ferrule (310) of the anchoring means.

27. Withdrawal ancillary according to Claim 3, **characterized in that** the rod (8) comprises a knurled head (170) provided with notches (270) of different dimensions, at least one of which comes into contact with a stop (280) integral with the body (4), after said rod has been adjusted in rotation in order to limit its translational travel.

## Patentansprüche

1. Hilfswerkzeug für ein elastisch verformbares Implantat (2, 3), das einen Körper (4), an den ein beweglicher Griff (5) angelenkt ist, und Bewegungsmittel (7, 14, 15; 110, 120, 130) aufweist, die eine Stange (8) zur Verformung des Implantats (2, 3) bewegen, **dadurch gekennzeichnet, dass** es außerdem eine Entkupplungsvorrichtung (19, 20, 21, 22, 26; 190, 200, 210, 230) aufweist, die einerseits in einer ersten entkuppelten Stellung erlaubt, die Stange (8) bezüglich des Körpers (4) frei drehbar und translationsbeweglich lassen, und andererseits in einer zweiten gekuppelten Stellung erlaubt, die Stange (8) bezüglich des Körpers (4) in Drehung und in Translation zu blockieren.

2. Hilfswerkzeug zum Einsetzen eines elastisch verformbaren Implantats (2, 3) für seine Befestigung im Knochen (47) eines Patienten nach Anspruch 1, **dadurch gekennzeichnet, dass** es Zugmittel (7, 14, 15) aufweist, welche die Stange (8) zur Verformung des Implantats (2, 3) verschieben.

3. Entnahme-Hilfswerkzeug zur Entnahme von Implantaten (2, 3) aus dem Knochen (47) nach Anspruch 1, **dadurch gekennzeichnet, dass** es Druckmittel (110, 120, 130) aufweist, welche die Stange (8) zur Verformung des Implantats (2) verschieben.

4. Hilfswerkzeug zum Einsetzen eines Implantats (2, 3) nach Anspruch 2, **dadurch gekennzeichnet, dass** der Körper (4) Mittel (27, 28, 29) zur Prüfung der Zugkraft aufweist, die zur Verformung des Implantats (2, 3) auf die Stange (8) ausgeübt wird.

5. Einsetz-Hilfswerkzeug nach Anspruch 2, **dadurch gekennzeichnet, dass** der Körper (4) eine erste Kammer (6), in der die Zugmittel (7, 14, 15) angeordnet sind, und eine zweite Kammer (9) aufweist, welche die Entkupplungsvorrichtung (19, 20, 21, 22, 26) aufnimmt.

6. Einsetz-Hilfswerkzeug nach Anspruch 2, **dadurch gekennzeichnet, dass** die Zugmittel aus einer ersten Platte (13) und einer zweiten Platte (14) bestehen, zwischen denen eine Feder (15) so zusammengedrückt ist, dass die zweite Platte (14) mit einem gekrümmten Umlenkhebel (7) zusammenwirkt, der vom beweglichen Griff (5) betätigt wird, um die zweite Platte (14) auf der Zugstange (8) derart abzustützen, dass sie bezüglich des Körpers (4) zurückweicht.

7. Einsetz-Hilfswerkzeug nach Anspruch 2, **dadurch gekennzeichnet, dass** die Entkupplungsvorrichtung einen Hebel (19), der von einem Vorsprung (20) blockiert werden kann, der elastisch an einem ortsfesten Griff (10) des Körpers (4) belastet ist, und eine Platte (22) aufweist, die mit einer Feder (26) verbunden ist, deren Spannung einstellbar ist, damit die Platte sich auf der Zugstange (8) unter einer Krafteinwirkung des Hebels abstützen kann, um von einer senkrechten entkuppelten Stellung in eine geneigte, gekuppelte Stellung überzugehen.

8. Einsetz-Hilfswerkzeug nach Anspruch 4, **dadurch gekennzeichnet, dass** die Prüfmittel aus einer fest mit dem Körper (4) verbundenen Nase (27), in der um die Stange (8) herum eine Druckfeder (28) vorgesehen ist, und einer Platte (29) bestehen, die fest mit einem Rohr (30) verbunden ist, das translationsbeweglich ist und in dem die Stange gleitet.

9. Einsetz-Hilfswerkzeug nach Anspruch 8, **dadurch gekennzeichnet, dass** die Platte (29) in einem Schlitz (36) mündet, der im oberen Bereich der Nase (27) ausgebildet ist und in dessen Nähe Markierungen (37) eingraviert sind, die es ermöglichen, die auf das Implantat (2, 3) ausgeübte Kraft zu überprüfen.

10. Einsetz-Hilfswerkzeug nach Anspruch 8, **dadurch gekennzeichnet, dass** das Rohr (30) an seinem freien Ende eine Führungsvorrichtung (31) aufweist, die einen inneren Gewindebereich besitzt, um einen Ansatz (32, 33) aufzunehmen, der die Formung des Implantats (2, 3) ermöglicht.

11. Einsetz-Hilfswerkzeug nach Anspruch 2, **dadurch gekennzeichnet, dass** die Zugstange (8) den Körper (4) von einer Seite zur anderen so durchquert, dass ihr sich auf der Seite der Entkupplungsvorrichtung befindliches Ende mit einem entfernbaren Kopf (11) zusammenwirkt, der die Einführung des Implantats (2, 3) in den Knochen (47) ermöglicht, während das andere Ende (34) einen geringeren Durchmesser als die Stange und einen Gewindebereich (35) aufweist, der ausgelegt ist, um sich ins Innere des Implantats (2, 3) einzuschrauben.

12. Einsetz-Hilfswerkzeug nach Anspruch 10, **dadurch gekennzeichnet, dass** der Ansatz (32) einen zylindrischen Körper (42) aufweist, von dem ein Teil ausgelegt ist, um sich in die Führungsvorrichtung (31) zu schrauben, während der andere Teil sich gegen das Implantat (2) anlegt.

13. Einsetz-Hilfswerkzeug nach Anspruch 12, **dadurch gekennzeichnet, dass** der Ansatz (32) in seinem inneren Bereich eine Feder (44) aufweist, die mit Druck auf eine Fadenführung (43) einwirkt.

14. Einsetz-Hilfswerkzeug nach Anspruch 10, **dadurch gekennzeichnet, dass** der Ansatz (32) einen zylindrischen Körper (45) aufweist, von dem ein Teil mit der Führungsvorrichtung (31) zusammenwirkt und der andere Teil eine konische Fläche (46) aufweist, die ausgelegt ist, um gegen das Implantat (3) in Anlage zu kommen.

15. Einsetz-Hilfswerkzeug nach Anspruch 10, **dadurch gekennzeichnet, dass** der Ansatz (32, 33) von der Zugstange (8) durchquert wird, damit das Ende (34) ausgelegt ist, um sich in das Implantat (2, 3) zu schrauben.

16. Einsetz-Hilfswerkzeug nach Anspruch 8, **dadurch gekennzeichnet, dass** die Nase (27) durch Schraubverbindung ein koaxial zum Rohr (30) liegendes Formgebungswerkzeug (39) aufnimmt, um das Implantat (3) in Form zu bringen.

17. Einsetz-Hilfswerkzeug nach Anspruch 16, **dadurch gekennzeichnet, dass** das Werkzeug (39) auf der Seite der Führungsvorrichtung (31) ein gekrümmtes Profil (41) aufweist.

18. Entnahme-Hilfswerkzeug nach Anspruch 3, **dadurch gekennzeichnet, dass** der Körper (4) um die Stange (8) herum frei drehbare Verankerungsmittel (290, 300, 310) aufweist, die ausgelegt sind, um sich innerhalb des zu entnehmenden Implantats (2) zu befestigen.

19. Entnahme-Hilfswerkzeug nach Anspruch 3, **dadurch gekennzeichnet, dass** der Körper (4) eine erste Kammer (6), in der die Druckmittel angeordnet sind, und eine zweite Kammer (9) aufweist, die die Entkupplungsvorrichtung aufnimmt.

20. Entnahme-Hilfswerkzeug nach Anspruch 3, **dadurch gekennzeichnet, dass** die Druckmittel aus einer ersten Platte (110) und einer zweiten Platte (120) bestehen, zwischen denen eine Feder (130) zusammengedrückt wird, wobei die erste Platte (110) mit dem beweglichen Griff (5) zusammenwirkt, um ihn auf der Stange (8) abzustützen, damit sie bezüglich des Körpers (4) vorwärts bewegt wird.

21. Entnahme-Hilfswerkzeug nach Anspruch 3, **dadurch gekennzeichnet, dass** die Entkupplungsvorrichtung eine Platte (190) aufweist, die von einem Vorsprung (200) blockiert werden kann, der elastisch auf dem ortsfesten Griff (10) des Körpers (4) belastet ist, wobei die Platte sich auf der Stange (8) mit Hilfe einer Feder (230) abstützt, die fest mit einer anderen senkrechten Platte (240) verbunden ist, die von der Stange durchquert wird, wobei die Platte (190) eine senkrechte entkuppelte Stellung bis eine geneigte gekuppelte Stellung einnehmen kann.

22. Entnahme-Hilfswerkzeug nach Anspruch 18, **dadurch gekennzeichnet, dass** die Verankerungsmittel aus einem gerändelten Element (290) bestehen, das fest mit einer Hülle (300) verbunden ist, die an ihrem freien Ende einen Gewindeansatz (310) trägt, der ausgelegt ist, um sich ins Innere des Implantats (2) einzuschrauben.

23. Entnahme-Hilfswerkzeug nach Anspruch 19, **dadurch gekennzeichnet, dass** die Verankerungsmittel aus einem gerändelten Element (290) bestehen, das fest mit einer Hülle (300) verbunden ist, die an ihrem freien Ende einen Gewindeansatz (310) trägt, der ausgelegt ist, um sich ins Innere des Implantats (2) zu schrauben, und dass das gerändelte Element (290) auf dem Körper (4) in der Verlängerung der ersten Kammer (6) entgegengesetzt zur zweiten Kammer (9) befestigt ist.

24. Entnahme-Hilfswerkzeug nach Anspruch 22, **dadurch gekennzeichnet, dass** das gerändelte Element (290) teilweise von einem Käfig (330) geschützt wird, der fest mit dem Körper (4) verbunden ist.

25. Entnahme-Hilfswerkzeug nach Anspruch 19, **dadurch gekennzeichnet, dass** die Stange (8) den Körper (4) von einer Seite zur anderen durchquert, um auf der Seite der zweiten Kammer (9) nach außen zu münden.

26. Entnahme-Hilfswerkzeug nach Anspruch 22, **dadurch gekennzeichnet, dass** die Stange (8) einen verjüngten Bereich (320) aufweist, der einen Drücker bildet, der innerhalb des Gewindeansatzes (310) der Verankerungsmittel gleitet.

27. Entnahme-Hilfswerkzeug nach Anspruch 3, **dadurch gekennzeichnet, dass** die Stange (8) einen gerändelten Kopf (170) mit Aussparungen (270) unterschiedlicher Abmessungen aufweist, von denen mindestens eine nach Drehregelung der Stange mit einem fest mit dem Kopf (4) verbundenen Anschlag (280) in Kontakt gelangt, um den Translationsweg der Stange zu begrenzen.
